## Europäisches Patentamt

## European Patent Office

## Office européen des brevets

(19)

(11) Veröffentlichungsnummer: **0 072 514**

**B1**

(12)

# EUROPÄISCHE PATENTSCHRIFT

(45) Veröffentlichungstag der Patentschrift:
13.08.86

(21) Anmeldenummer: 82107162.8

(22) Anmeldetag: 07.08.82

(51) Int. Cl.⁴: **C 07 B 39/00 //**
C07C37/62, C07C17/12

(54) Verfahren zur Halogenierung von organischen Verbindungen.

(30) Priorität: 19.08.81 DE 3132692

(43) Veröffentlichungstag der Anmeldung:
23.02.83 Patentblatt 83/8

(45) Bekanntmachung des Hinweises auf die Patenterteilung:
13.08.86 Patentblatt 86/33

(84) Benannte Vertragsstaaten:
BE CH DE FR GB IT LI NL

(56) Entgegenhaltungen:
DE-A-1 667 189
DE-A-2 063 601

(73) Patentinhaber: BAYER AG, Konzernverwaltung RP Patentabteilung, D-5090 Leverkusen 1 Bayerwerk (DE)

(72) Erfinder: Judat, Helmut, Dr., Oskar- Erbslöh- Strasse 44a, D-4018 Langenfeld (DE)
Erfinder: Schnegg, Ulrich, Dr., Ulrich- von- Hassell- Strasse 13, D-5090 Leverkusen 1 (DE)
Erfinder: Wedemeyer, Karlfried, Dr., Bilharzstrasse 7, D-5000 Köln 80 (DE)

EP 0 072 514 B1

LIBER, STOCKHOLM 1986

**Beschreibung**

Die Erfindung betrifft ein Verfahren zur Halogenierung von organischen Verbindungen in der flüssigen Phase mit gasförmigen Halogenierungsmitteln.

Die Halogenierung von organischen Verbindungen in der flüssigen Phase mit gasförmigen Halogenierungsmitteln, wie Chlor oder Brom, ist seit langem bakannt (vgl. z.B. Houben-Weyl, Methoden der organischen Chemie, Band V/3, Seite 516 (1962)).

Ebenfalls bekannt ist die Abhängigkeit der Selektivität zwischen der Mono- und Dihalogenierung von der Blasengröße des eingesetzten, gasförmigen Halogenierungsmittels, wenn es sich un schnelle Reaktionen, die an der Grenzfläche der Gas- und Flüssigphase ablaufen, handelt. So ist aus J. Chem. Eng. (Japan), Vol. 3, No. 1, Seite 79 ff (1970) bekannt, daß die Selektivitätsabhängigkeit bei der Chlorierung von p-Kresol ein Stofftransportproblem in der Phasentrennschicht ist. Es wurde dabei gefunden, daß die Selektivität bezüglich der Monochlorierung verbessert werden kann, wenn der Blasendurchmesser des zugegebenen, gasförmigen Chlorierungsmittels vergrößert wird. Das beste Ergebnis hinsichtlich der Selektivität wird erzielt bei großen Gasblasen mit einem Durchmesser von größer als 2,5 mm.

Aus der DE—OS—20 63 601 ist ein Verfahren zur kontinuierlichen Herstellung von linearen Monochloralkenen mit mindestens 9 C-Atomen durch Chlorierung der Alkane in flüssiger Phase mit gasförmigem Chlor bei erhöhter Temperatur und in Abwesenheit von Katalysatoren bekannt. Auf Seite 3 der genannten DE—OS wird erwähnt, daß das Chlor bei der Halogenierung von Chloralkanen mit einer Geschwindigkeit von mindestens 50 m/s zugeführt wird. Die hohe Gaseinleitgeschwindigkeit dient offensichtlich einer besseren Durchmischung der Phasen in den einzelnen Reaktionsabschnitten. Außerdem wird im ersten Absatz auf Seite 4 der genannten DE—OS ausgeführt, daß die Trennwände ein Vermischen der Reaktionsflüssigkeiten, eine lokal auftretende hohe Chlorkonzentration und dadurch die Mehrfachsubstitution vermeiden.

Weiterhin ist aus der DE—OS 1 667 189 ein Verfahren zur kontinuierlichen Durchführung von Reaktionen, an denen Gase und Flüssigkeiten beteiligt sind und Gas und Flüssigkeit einen vertikalen, lang gestreckten Reaktor von unten nach oben durchströmen, bekannt, das dadurch gekennzeichnet ist, daß das Gas in die Flüssigkeit mit solcher Geschwindigkeit durch einen Reaktor, der durch horizontale mit Öffnungen versehene Zwischenböden in einzelne Reaktionskammern unterteilt ist, geleitet werden, daß die lineare Geschwindigkeit des Gases in den Öffnungen 1 bis 50 m/s beträgt. Ziel des in der DE—OS 1 667 189 beschriebenen Verfahrens ist die Steigerung der Raum-Zeit-Ausbeuten von Reaktionen, an denen Gase und Flüssigkeiten beteiligt sind und bei deren Durchführung Gas und Flüssigkeit einen vertikalen, lang gestreckten Reaktor von unten nach oben durchströmen.

Es wurde nun ein Verfahren zur Halogenierung von organischen Verbindungen, bei denen eine Mehrfachhalogenierung möglich ist und die bei der Reaktion mit gasförmigen Halogenierungsmitteln einen Selektivitätsunterschied bezüglich der Erst- und Zweithalogenierung aufweisen, wenn diese in Form von Gasblasen mit einem mittleren Durchmesser von ca. 3 mm und einem mittleren Durchmesser von ca. 1 mm in das Reaktionsgemisch eingeleitet werden, in der flüssigen Phase gefunden, das dadurch gekennzeichnet ist, daß man das gasförmige Halogenierungsmittel mit einer solchen Eintrittsgeschwindigkeit in das Reaktionsgefäß eindosiert, daß sich an der Eintrittsstelle des Halogenierungsmittels ein Gesstrahl im Reaktionsgemisch ausbildet und die Bildung von einzelnen Gasblasen an der Eintrittsstelle vermieden wird.

Es hat sich gezeigt, daß sich beim Einleiten des gasförmigen Halogenierungsmittels in das Reaktionsgefäß dann ein Gasstrahl im Reaktionsgemisch ausbildet und die Bildung von einzelnen Gasblasen an der Eintrittsstelle des Halogenierungsmittels vermieden wird, wenn die Geschwindigkeit des gasförmigen Halogenierungsmittels an der Eintrittsstelle größer als 7 m/s ist. Durch eine Erhöhung der Eintrittsgeschwindigkeit kann die Selektivität noch verbessert werden. Nach oben hin ist die Eintrittsgeschwindigkeit des gasförmigen Halogenierungsmittels praktisch nur durch technische Gegebenheiten begrenzt, beispielsweise durch den erforderlichen Vordruck zur Erzeugung einer hohen Eintrittsgeschwindigkeit. Ein sinnvoller Kompromiß zwischen technischen Aufwand und Selektivitätsverbesserung ist dann gewährleistet, wenn die Eintrittsgeschwindigkeit des gasförmigen Halogenierungsmittels in einem Bereich von etwa 9 bis 100 m/s, bevorzugt 15 bis 50 m/s, liegt.

Das erfindungsgemäße Verfahren läßt sich in vorteilhafter Weise bei solchen organischen Verbindungen anwenden, bei denen eine Mehrfachhalogenierung möglich ist und die bei der Reaktion mit gasförmigen Halogenierungsmitteln einen Selektivitätsunterschied bezüglich der Erst- und Zweithalogenierung aufweisen. Um einen Selektivitätsunterschied bei einer Mehrfachhalogenierung einer organischen Verbindung festzustellen, geht man z.B. so vor, daß man in das Reaktionsgemisch das Halogenierungsmittel einmal in Form von Gasblasen mit einem mittleren Durchmesser von etwa 3 mm und einmal in Form von Gasblasen mit einem mittleren Durchmesser von etwa 1 mm unter sonst gleichen Reaktionsbedingungen einleitet. Wird bei der Halogenierung mit Gasblasen, die einen mittleren Durchmesser von etwa 3 mm besitzen, eine Selektivitätsverbesserung bezüglich der Ersthalogenierung erreicht, dann sind die dabei eingesetzten organischen Verbindungen für das erfindungsgemäße Verfahren geeignet. Das bedeutet, daß sich dann bei der Halogenierung dieser organischen Verbindungen nach dem erfindungsgemäßen Verfahren nochmals eine beträchtliche Selektivitätsverbesserung und damit eine Ausbeuteverbesserung hinsichtlich des Ersthalogenierungsproduktes erzielen läßt. Zum Beispiel erhöht

2

sich dabei die Ausbeute an Monohalogenverbindung, wenn man von einer noch nicht halogenierten organischen Verbindung ausgeht. Bei den für das erfindungsgemäße Verfahren besonders geeigneten organischen Verbindungen sind die Halogenierungsgeschwindigkeiten der Erst- und Zweit- bzw. Mehrfachhalogenierung so hoch, daß sie in der flüssigkeitseitigen Grenzschicht ablaufen. Das bedeutet, daß die stationäre Konzentration des Halogenierungsmittels im Reaktionsgemisch sehr gering ist und im allgemeinen unterhalb 1 Gew.-%, bevorzugt unterhalb 0,05 bis 0,1 Gew.-%, bezogen auf das Reaktionsgemisch, liegt.

Als organische Verbindungen können sowohl aliphatische als als auch aromatische, zur Mehrfachhalogenierung fähige Kohlenwasserstoffe eingesetzt werden, wie Hydroxyaromaten, Aminaromaten, Alkylaromaten, Arylaromaten, Alkyl- oder Aryloxyaromaten, Ketone, Ester, Nitrile sowie Di- und Polyene.

In das erfindungsgemäße Verfahren können die organischen Verbindungen sowohl in Substanz als auch gelöst oder dispergiert in einem inerten organischen Lösungs- und/oder Verdünnungsmittel eingesetzt werden. Als inerte organische Lösungs- und/oder Verdünnungsmittel kommen z.B. in Frage: Tetrachlorkohlenstoff, Methylenchlorid, Dichlorethan, Trichlorethan, Tetrachlorethan, Chlorbenzol, Essigsäure, Dioxan, Dimethylformamid und/oder Wasser, bevorzugt Tetrachlorkohlenstoff, Dichlorethan, Tetrachlorethan und/oder Essigsäure.

Die Menge der Lösungs- und/oder Verdünnungsmittel kann in weiten Bereichen schwanken und hängt vor allem von der Löslichkeit der zu halogenierenden organischen Verbindung in dem entsprechenden Lösungsmittel und von der gewünschten Raum/Zeit-Ausbeute ab. Die jeweils günstigste Menge an Lösungs- und/oder Verdünnungsmittel kann leicht durch Vorversuche ermittelt werden.

Als Halogenierungsmittel können in das erfindungsgemäße Verfahren Chlor, Brom oder Jod, bevorzugt Chlor oder Brom, besonders bevorzugt Chlor, eingesetzt werden. Die Halogenierungsmittel werden dabei gasförmig in der zuvor beschriebenen Weise in das Reaktionsgemisch eingeleitet. Die Menge des eingesetzten Halogenierungsmittels richtet sich nach dem gewünschten Umsatz der eingesetzten organischen Verbindungen und kann durch Vorversuche leicht bestimmt werden. Zum Beispiel erhält man bei schnellen Reaktionen maximale Ausbeuten an Monohalogenverbindung, wenn man etwa 1,0 Mol Halogenierungsmittel pro Mol organischer Verbindung einsetzt. Es kann aber auch zweckmäßig sein, weniger als 1 Mol Halogenierungsmittel, beispielsweise 0,6 bis 0,9 Mol Halogenierungsmittel, pro Mol organische Verbindung, einzusetzen und das noch nicht umgesetzte Ausgangsmaterial in den Halogenierungsprozeß zurückzuführen. Es ist jedoch auch möglich, mehr als 1 Mol Halogenierungsmittel, z.B. 1,1 bis 1,4 Mol. pro Mol organischer Verbindung einzusetzen.

Das Halogenierungsmittel kann in das erfindungsgemäße Verfahren auch in Abmischung mit anderen inerten Gasen wie Stickstoff, Kohlendioxid, Edelgase, wie Helium oder Argon, und/oder Halogenwasserstoffs, wie Chlorwasserstoff oder Bromwasserstoff, und/oder Dämpfen, wie Wasserdampf und/oder Lösungsmitteldämpfe, wie Methylenchloriddampf, eingesetzt werden.

Die Temperaturen können in weiten Bereichen schwanken, wobei eine Begrenzung der Reaktionstemperatur nach unten und nach oben hin dadurch gegeben ist, daß bei sehr tiefen Temperaturen die Reaktionsgeschwindigkeiten der Halogenierungen im Vergleich zum Stofftransport zu langsam werden und damit eine Selektivitätsverbesserung nicht mehr erzielt werden kann und bei sehr hohen Temperaturen eine flüssige Phase nicht mehr aufrechterhalten werden kann. Die günstigste Reaktionstemperatur kann jedoch durch einfache Vorversuche leicht ermittelt werden. Im allgemeinen führt man die erfindungsgemäße Halogenierung bei etwa 0 bis 150°C, bevorzugt bei 40 bis 100°C, durch.

Das erfindungsgemäße Verfahren kann je nach Bedarf bei Normaldruck, Unterdruck oder bei Überdruck durchgeführt werden. Bevorzugt wird das Verfahren bei Normaldruck durchgeführt.

Die Einleitung des Halogenierungsmittels in das Reaktionsgemisch kann mit den üblichen Gaseinleitungsvorrichtungen bewerkstelligt werden. Zum Beispiel ist es möglich, die Einleitung des Halogenierungsmittels mit Hilfe eines Rohres, einer Lochblende oder eines Begasungsringes, der mit mehreren kleinen Bohrungen versehen ist, durchzuführen.

Das erfindungsgemäße Verfahren kann sowohl kontinuierlich als auch diskontinuierlich durchgeführt werden.

Die Möglichkeit einer technischen Durchführung des erfindungsgemäßen Verfahrens sei im folgenden anhand der Chlorierung von Phenol veranschaulicht.

Zu einer in einer Blasensäule mit Rückflußkühler vorgelegten Lösung von Phenol in Tetrachlorkohlenstoff wird durch ein Einleitungsrohr, welches an seinem Ende zu einer Spitze ausgezogen ist, Chlor mit einer Eintrittsgeschwindigkeit von 15 bis 25 m/s in die Blasensäule eindosiert. Nachdem die dem gewünschten Umsatz entsprechende Menge an Chlor eingeleitet worden ist, wird der Tetrachlorkohlenstoff abdestilliert und der Rückstand in bekannter Weise durch fraktionierte Destillation aufgearbeitet.

Gemäß einer anderen Verfahrensvariante (kontinuierliches Verfahren) werden z.B. die Lösung von Phenol in Tetrachlorkohlenstoff und das Chlor in dem dem gewünschten Umsatz entsprechenden Verhältnis simultan in eine einstufige Blasensäule eindosiert. Die Eintrittsgeschwindigkeit des Chlorgases beträgt ca. 20 m/s. Durch die freiwerdende Reaktionswärme erhitzt sich das Reaktionsgemisch dabei auf Siedetemperatur. Die Wärme wird über Siedekühlung des rückfließenden Lösungsmittels abgeführt. Das aus dem Reaktor direkt in eine Destillationskolonne fließende Produktgemisch wird durch Destillation vom Lösungsmittel befreit. Zurück bleibt ein Chlorphenolgemisch. Das bei der Reaktion entstehende Abgas, das größtenteils aus Chlorwasserstoff besteht, wird ebenfalls in die Destillationskolonne eingeleitet und dort von anhaftenden hochsiedenden organischen Produkten befreit. Das dann aus der Destillationskolonne entweichende Abgas, das neben Chlorwasserstoff nahezu ausschließlich nur noch die dem Dampfdruck entsprechende Menge an Tetrachlorkohlenstoff enthält, wird in einem adiabatisch arbeitenden Wäscher mit Wasser gewaschen, wobei im Sumpf des Wäschers eine hochreine Salzsäure anfällt, die in dieser Form für weitere Reaktionen direkt eingesetzt werden kann und daher keine Entsorgungsprobleme aufwirft. Das rohe Chlorphenolgemisch wird in bekannter Weise im Vakuum in die reinen Komponenten aufdestilliert.

Durch das erfindungsgemäße Verfahren kann die Selektivität von Halogenierungsreaktionen, die in der Gas-/Flüssig-Phase ablaufen, wasentlich verbessert werden. Die hohe Selektivität, mit der die Halogenierung abläuft, bewirkt zudem eine Reihe weiterer Vorteile, wie etwa eine höhere Ausbeute bezüglich des Ersthalogenierungsproduktes, weniger unerwünschte Nebenprodukte, was mit einem geringeren Kostenanfall bei der Beseitigung der Nebenprodukte einhergeht, die Möglichkeit bei höheren Temperaturen zu Halogenieren, wodurch eine einfachere Ableitung der Reaktionswärme durch Kühlwasser statt der teuren Solekühlung möglich wird. Außerdem kann die Halogenierung in wesentlich konzentrierterer Lösung durchgeführt werden, ohne daß gegenüber dem herkömmlichen Verfahren mehr Nebenprodukte anfallen. Dadurch ist eine deutlich bessere Raum/Zeit-Ausbeute zu erzielen, die in die Wirtschaftlichkeitsbetrachtung einer technischen Anlage stark eingeht.

Bei dem erfindungsgemäßen Verfahren überrascht besonders, daß die genannten Vorteile dadurch erreicht werden, daß man eine Blasenbildung bei der Einleitung des gasförmigen Halogenierungsmittels in das Reaktionsgemisch gänzlich vermeidet, obwohl gemäß dem Stand der Technik (vgl. z.B. J. chem. Eng. (Japan), Vol. 3, No. 1, S. 79 ff (1970)) die Selektivität bei der Halogenierung von organischen Verbindungen, bei denen eine Mehrfachhalogenierung möglich ist, gesteigert wird, wenn man den Blasendurchmesser des gasförmig eingeleiteten Halogenierungsmittels vergrößert.

Die nach dem erfindungsgemäßen Verfahren herstellbaren halogenierten organischen Verbindungen finden teils als Endprodukte, zum überwiegenden Teil jedoch als Zwischenprodukte für z.B. Pflanzenschutzmittel, Pharmazeutika und Farbstoffe Verwendung.

Die nachfolgenden Beispiele sollen das erfindungsgemäße Verfahren verdeutlichen.

Beispiel 1 (Vergleichsbeispiel)

600 ml einer 4,66-molaren Lösung von m-Kresol in 1,2-Dichlorethan werden in einer Blasensäule unter Temperung von außen auf 50°C gebracht und bei dieser Temperatur über eine Fritte D 1 (Porengröße 100—160 µm) am Boden der Blasensäule mit 179 g Chlorgas (durchschnittliche Blasengröße kleiner als 1 mm) innerhalb von ca. 3 Stunden begast. Anschließend wird abgekühlt und das Reaktionsgemisch gaschromatographisch untersucht (100%-Methode; s. Rudolf Kaiser, Chromatographie in der Gasphase; Band 4, Quantitative Auswertung 2. Teil, S. 208; B) Hochschultaschenbücher, Mannheim/Zürich 1969). Man erhält ein Gemisch aus:

| | |
|---|---|
| 3-Hydroxytoluol | 28,1% |
| 4-Chlor-3-hydroxytoluol | 21,2% |
| 6-Chlor-3-hydroxytoluol | 35,3% |
| 4,6-Dichlor-3-hydroxytoluol | 13,6% |

Rest: nicht identifizierte Produkte

Beispiel 2 (Vergleichsbeispiel)

Beispiel 1 wird wiederholt, aber die Fritte D 1 durch eine Rohr mit 5 mm Durchmesser ersetzt. Der mittlere Durchmesser der Chlorblasen ist dabei größer als 3 mm. Die gaschromatographische Analyse des Rohprodukts ergibt:

| | |
|---|---|
| 3-Hydroxytoluol | 19,7% |
| 4-Chlor-3-hydroxytoluol | 28,3% |
| 4-Chlor-3-hydroxytoluol | 46,4% |
| 4,6-Dichlor-3-hydroxytoluol | 4,6% |

Rest: nicht identifizierte Produkte

Beispiel 3

Beispiel 1 wird wiederholt, aber die Fritte D 1 durch ein zu einer Spitze von Durchmesser 0,8 mm ausgezogenes Rohr ersetzt. Die Eintrittsgeschwindigkeit des Chlorgases liegt bei ca. 20 m/s. Die gaschromatographische Analyse das Produktgemisches nach 1,5 Stunden Einleitungszeit ergibt dann:

4

# 0 072 514

| | |
|---|---|
| 3-Hydroxytoluol | 13,0% |
| 4-Chlor-3-hydroxytoluol | 29,1% |
| 6-Chlor-3-hydroxytoluol | 53,0% |
| 4,6-Dichlor-3-hydroxytoluol | 3,6% |

Rest: nicht identifizierte Produkte

Die Selektivitätsverbesserung durch das neue Verfahren wird besonders deutlich in den Verhältniszahlen von nichtchloriertem zu mono-:dichloriertem Material von

0,50:1,00:0,25 in Beispiel 1
0,26:1,00:0,06 in Beispiel 2
0,16:1,00:0,04 in Beispiel 3.

Beispiel 4 (Vergleichsbeispiel)

Beispiel 1 wird wiederholt, aber statt einer 4,66-molaren Lösung von m-Kresol in Dichlorethan wird eine 4,23-molare Lösung von Phenol in Tetrachlorkohlenstoff eingesetzt.

Man erhält nach Einleiten von 200 g Chlor (Gasblasen mit einem mittleren Durchmesser von kleiner als 1 mm) ein Rohgemisch, das nach geschromatographischer Analyse folgende Zusammensetzung aufweist:

| | |
|---|---|
| Phenol | 11,8% |
| 2-Chlorphenol | 34,3% |
| 4-Chlorphenol | 37.1% |
| 2,4-Dichlorphenol | 15,0% |
| 2,6-Dichlorphenol | 1,1% |

Rest: nicht identifizierte Produkte

Beispiel 5 (Vergleichsbeispiel)

Beispiel 2 wird wiederholt, aber wiederum die m-Kresollösung durch die Phenollösung ersetzt. Man erhält:

| | |
|---|---|
| Phenol | 6,8% |
| 2-Chlorphenol | 44,1% |
| 4-Chlorphenol | 42,9% |
| 2,4-Dichlorphenol | 5,5% |
| 2,6-Dichlorphenol | 0,4% |

Rest: nicht identifizierte Produkte

Beispiel 6

Beispiel 3 wird mit der Phenollösung von Beispiel 4 wiederholt. Man erhält:

| | |
|---|---|
| Phenol | 8,5% |
| 2-Chlorphenol | 42,4% |
| 4-Chlorphenol | 46,6% |
| 2,4-Dichlorphenol | 2,1% |
| 2,6-Dichlorphenol | 0,1% |

Rest: nicht identifizierte Produkte

Beispiel 6 zeigt im Vergleich zu den Beispielen 4 und 5 eine wesentliche Abnahme an Dichlorphenolen.

5

Beispiele 7 bis 11

Die Beispiele 1 bis 3 werden wiederholt, aber statt der m-Kresollösung wird eine 4,66-molare Lösung von p-Kresol in Tetrachlorkohlenstoff eingesetzt und die Temperatur in den Beispielen 10 (Ausführung analog Beispiel 2) und 11 (Ausführung analog Beispiel 3) wird auf 20°C gehalten. Man erhält Rohprodukte, deren Zusammensetzung in der nachfolgenden. Tabelle wiedergegeben wird.

TABELLE

Zusammensetzung der Rohprodukte aus den Beispielen 7 bis 11, angegeben in Gew.-%:

| Beispiel | 7* | 8** | 9*** | 10** | 11*** |
|---|---|---|---|---|---|
| 4-Hydroxytoluol | 7,4 | 5,9 | 5,3 | 9,8 | 7,4 |
| 3-Chlor-4-hydroxytoluol | 77,5 | 82,0 | 87,1 | 79,7 | 82,8 |
| 3,5-Dichlor-4-hydroxytoluol | 6,3 | 3,2 | 0,9 | 1,6 | 0,5 |

Rest: nicht identifierte Produkte
* analog Beispiel 1 (Vergleichsbeispiel)
** analog Beispiel 2 (Vergleichsbeispiel)
*** analog Beispiel 3

Wiederum wird die Selektivitätsverbesserung durch das neue Verfahren besonders deutlich in den Verhältniszahlen von nichtchloriertem zu mono-:dichloriertem Material von

0,10:1:0,08 in Beispiel 7
0,07:1:0,04 in Beispiel 8
0,06:1:0,01 in Beispiel 9
0,12:1:0,02 in Beispiel 10
0,09:1:0,01 in Beispiel 11.

Beispiele 12 bis 14

Die Beispiele 1 bis 3 werden wiederholt, aber statt der Lösung von m-Kresol wird m-Xylol in Substanz eingesetzt, 2,6-Gew.-% FeCl$_3$ werden als Katalysator zugegeben, und es wird bei 70°C chloriert. Nach Einleiten von 90 Mol-% Chlor erhält man Rohprodukts, deren Zusammensetzung in der nachfolgenden Tabelle wiedergegeben wird.

TABELLE

Zusammensetzung der Rohprodukte aus den Beispielen 12 bis 14, angegeben in Gew.-%:

| Beispiel | 12* | 13** | 14*** |
|---|---|---|---|
| m-Xylol | 31,6 | 27,2 | 23,6 |
| 4-Chlor-m-xylol | 43,4 | 56,8 | 63,7 |
| 4,6-Dichlor-m-xylol | 14,0 | 11,6 . | 10,7 |

Rest: nicht identifizierte Produkte
* analog Beispiel 1 (Vergleichsbeispiel)
** analog Beispiel 2 (Vergleichsbeispiel)
*** analog Beispiel 3.

Beispiel 15

In eine 2 l fassende Blasensäule mit Rückflußkühler, Gaseinleitungsdüse (1,5 mm Durchmesser), Gasableitungsrohr, Tauchrohr und Überlaufrohr werden kontinuierlich 1000 ml pro Stunde einer 33 gew.-%igen Phenollösung in Tetrachlorkohlenstoff über das Tauchrohr eingepumpt. Über die Gaseinleitungsdüse werden unter Rückflußsieden 360 g Chlor pro Stunde eingeleitet (die Eintrittsgeschwindigkeit des Chlors beträgt ca. 18 m/s. Über das Überlaufrohr verläßt kontinuierlich ein Strom rohes Chlorphenol gelöst in Tetrachlorkohlenstoff die Blasensäule. Die Rohlösung läuft direkt in die Mitte einer 1,5 m langen Silbermantelkolonne, die mit Füllkörpern gefüllt ist. Das Abgas wird über den wassergekühlten Rückflußkühler vom größten Teil des Tetrachlorkohlenstoffs befreit und dann ebenfalls in die Kolonne eingespeist. Im Sumpf der Kolonne fallen 620 g pro Stunde rohes Chlorphenol an, während sich am Kopf

6

**0 072 514**

der Kolonne über ein Kühlsystem mit Solekülung aus dem Abgas 873 g Tetrachlorkohlenstoff abscheiden. Das rohe Chlorphenolgemisch enthält nach einer gaschromatographischen Analyse:

| | |
|---|---|
| Phenol | 5,3% |
| 2-Chlorphenol | 40,4% |
| 4-Chlorphenol | 39,8% |
| 2,4-Dichlorphenol | 13,6% |
| 2,6-Dichlorphenol | 0,9% |

Rest: nicht identifizierte Produkte

Das gekühlte Abgas wird einer adiabatischen Wäsche zugeleitet, bei der in einer 1,5 m hohen Glasfüllkörperkolonne 420 g Wasser pro Stunde dem Gasgemisch entgegenströmen. Am Kopf der Kolonne wird eine Mischung aus rückfließendem Wasser und 15 g/h Tetrachlorkohlenstoff abgenommen. Aus dem Sumpf der Kolonne werden 600 g/h 30 gew.-%iger Salzsäure entnommen, die einen Gesamtkohlenstoffgehalt (organisch gebundener Kohlenstoff plus anorganischer Kohlenstoff) von 6 ppm aufweisen.

**Patentansprüche**

1. Verfahren zur Halogenierung von organischen Verbindungen, bei denen eine Mehrfachhalogenierung möglich ist und die bei der Reaktion mit gasförmigen Halogenierungsmitteln einen Selektivitätsunterschied bezüglich der Erst- und Zweithalogenierung aufweisen, wenn diese in Form von Gasblasen mit einem mittleren Durchmesser von ca. 3 mm und einem mittleren Durchmesser von ca. 1 mm in das Reaktionsgemisch eingeleitet werden, in der flüssigen Phase, dadurch gekennzeichnet, daß man das gasförmige Halogenierungsmittel mit einer solchen Eintrittsgeschwindigkeit in das Reaktionsgefäß eindosiert, daß sich an der Eintrittsstelle des Halogenierungsmittels ein Gasstrahl im Reaktionsgemisch ausbildet und die Bildung von einzelnen Gasblasen an der Eintrittsstelle vermieden wird.

2. Verfahren nach Anspruch 1, dadurch gekennzeichnet, daß man das gasförmige Halogenierungsmittel mit einer Eintrittsgeschwindigkeit von größer als 7 m/s in das Reaktionsgefäß eindosiert.

3. Verfahren nach Anspruch 1, dadurch gekennzeichnet, daß man das gasförmige Halogenierungsmittel mit einer Eintrittsgeschwindikeit von 9 bis 100 m/s in das Reaktionsgefäß eindosiert.

4. Verfahren nach Anspruch 1, dadurch gekennzeichnet, daß man das gasförmige Halogenierungsmittel mit einer Eintrittsgeschwindigkeit von 15 bis 50 m/s in das Reaktionsgefäß eindosiert.

5. Verfahren nach Ansprüchen 1 bis 4, dadurch gekennzeichnet, daß man als Halogenierungsmittel Chlor, Brom oder Jod einsetzt.

6. Verfahren nach Ansprüchen 1 bis 4, dadurch gekennzeichnet, daß man als Halogenierungsmittel Chlor einsetzt.

7. Verfahren nach Ansprüchen 1 bis 6, dadurch gekennzeichnet, daß man die Halogenierungsmittel in Abmischung mit inerten Gasen oder Dämpfen einsetzt.

8. Verfahren nach Ansprüchen 1 bis 7, dadurch gekennzeichnet, daß man die Halogenierungsmittel in Abmischung mit Stickstoff, Kohlendioxid, Helium, Argon, Chlorwasserstoff, Bromwasserstoff, Wasserdampf und/oder Methylenchloriddampf einsetzt.

9. Verfahren nach Ansprüchen 1 bis 8, dadurch gekennzeichnet, daß man das Verfahren bei Temperaturen von 0 bis 150°C durchführt.

10. Verfahren nach Ansprüchen 1 bis 8, dadurch gekennzeichnet, daß man das Verfahren bei Temperaturen von 40 bis 100°C durchführt.

**Revendications**

1. Procédé d'halogénation de composés organiques dans lesquels une polyhalogénation est possible et qui présentent dans la réaction avec des agents d'halogénation gazeux une différence de sélectivité quant à la première et à la seconde halogénation quand ceux-ci sont alimentés sous forme de bulles gazeuses ayant un diamètre moyen d'environ 3 mm et un diamètre moyen d'environ 1 mm dans le mélange de réaction, dans la phase liquide, caractérisé en ce qu'on alimente l'agent d'halogénation gazeux dans le récipient de réaction à une vitesse d'admission telle qu'il se constitue à l'endroit de l'admission de l'agent d'halogénation un jet gazeux dans le mélange de réaction et en ce que la formation de bulles gazeuses individuelles est évitée à l'endroit de l'admission.

2. Procédé selon la revendication 1, caractérisé en ce qu'on alimente l'agent d'halogénation gazeux à une vitesse d'admission supérieure à 7 m/s dans le récipient de réaction.

3. Procédé selon la revendication 1, caractérisé en ce qu'on alimente l'agent d'halogénation gazeux à une vitesse d'admission de 9 à 100 m/s dans le récipient de réaction.

7

## 0 072 514

4. Procédé selon la revendication 1, caractérisé en ce qu'on alimente l'agent d'halogénation gazeux à une vitesse d'admission de 15 à 50 m/s dans le récipient de réaction.

5. Procédé selon les revendications 1 à 4, caractérisé en ce qu'on utilise comme agent d'halogénation du chlore, du brome ou de l'iode.

6. Procédé selon les revendication 1 à 4, caractérisé en ce qu'on utilise du chlore comme agent d'halogénation.

7. Procédé selon les revendications 1 à 6, caractérisé en ce qu'on utilise l'agent d'halogénation en mélange avec des gaz ou des vapeurs inertes.

8. Procédé selon les revendications 1 à 7, caractérisé en ce qu'on utilise l'agent d'halogénation en mélange avec de l'azote, du dioxyde de carbone, de l'hélium, de l'argon, de l'acide chlorhydrique, de l'acide bromhydrique, de la vapeur d'eau et/ou de la vapeur de chlorure de méthylène.

9. Procédé selon les revendications 1 à 8, caractérisé en ce qu'on exécute le procédé à des températures de 0 à 150°C.

10. Procédé selon les revendications 1 à 8, caractérisé en ce qu'on exécute le procédé à des températures de 40 à 100°C.

### Claims

1. Process for the halogenation of organic compounds in which polyhalogenation is possible and which exhibit a difference in selectivity with respect to the first and second halogenation when reacted with gaseous halogenating agents when the latter are introduced into the reaction mixture in the form of gas bubbles having an average diameter of about 3 mm and an average diameter of about 1 mm, in the liquid phase, characterised in that the gaseous halogenating agent is metered into the reaction vessel at such an entry velocity that a gas jet is formed in the reaction mixture at the point of entry of the halogenating agent and the formation of individual gas bubbles at the point of entry is prevented.

2. Process according to Claim 1, characterised in that the gaseous halogenating agent is metered into the reaction vessel at an entry velocity of greater than 7 m/s.

3. Process according to Claim 1, characterised in that the gaseous halogenating agent is metered into the reaction vessel at an entry velocity of 9 to 100 m/s.

4. Process according to Claim 1, characterised in that the gaseous halogenating agent is metered into the reaction vessel at an entry velocity of 15 to 50 m/s.

5. Process according to Claims 1 to 4, characterised in that the halogenating agent employed is chlorine, bromine or iodine.

6. Process according to Claims 1 to 4, characterised in that chlorine is employed as the halogenating agent.

7. Process according to Claims 1 to 6, characterised in that the halogenating agents are employed as a mixture with inert gases or vapours.

8. Process according to Claims 1 to 7, characterised in that the halogenating agents are employed as a mixture with nitrogen, carbon dioxide, helium, argon, hydrogen chloride, hydrogen bromide, water vapour and/or methylene chloride vapour.

9. Process according to Claims 1 to 8, characterised in that the process is carried out at temperatures from 0 to 150°C.

10. Process according to Claims 1 to 8, characterised in that the process is carried out at temperatures from 40 to 100°C.

8